# EUROPÄISCHE PATENTSCHRIFT

(11) **EP 3 997 062 B1**
(45) Veröffentlichungstag und Bekanntmachung des Hinweises auf die Patenterteilung: **26.02.2025**
(21) Anmeldenummer: 20735517.3
(22) Anmeldetag: 25.06.2020
(51) Int. Cl.: C07C 277/08, C07C 279/14, A23K 20/142

(54) **METASTABILE KRISTALLMODIFIKATION UND VERFAHREN ZU DEREN HERSTELLUNG (I)**
METASTABLE CRYSTAL MODIFICATION AND METHOD FOR THE PRODUCTION THEREOF (I)
MODIFICATION CRISTALLINE MÉTASTABLE ET PROCÉDÉ DE PRODUCTION ASSOCIÉ (I)

(30) Priorität: 12.07.2019 DE 102019118893; 12.07.2019 DE 102019118894
(43) Veröffentlichungstag der Anmeldung: 18.05.2022
(73) Patentinhaber: Alzchem Trostberg GmbH, 83308 Trostberg (DE)
(72) Erfinder: GÜTHNER, Thomas, 83308 Trostberg (DE); THALHAMMER, Franz, 83308 Trostberg (DE); SANS, Jürgen, 83308 Trostberg (DE)
(74) Vertreter: Weickmann & Weickmann PartmbB
(86) Internationale Anmeldenummer: PCT/EP2020/067837
(87) Internationale Veröffentlichungsnummer: WO 2021/008843

(56) Entgegenhaltungen:
- CN-A- 101 525 305
- DE-C- 964 590
- US-A- 2 654 779
- G. P. JONES ET AL: "Conformations of GABA analogues. I: Crystal and molecular structure of guanidinoacetic acid", JOURNAL OF CRYSTAL AND MOLECULAR STRUCTURE, vol. 9, no. 5, 1 October 1979 (1979-10-01), US, pages 273 - 279, XP055716473, ISSN: 0308-4086, DOI: 10.1007/BF01320842
- S. GUHA: "The crystal and molecular structure of glycocyamine", ACTA CRYSTALLOGRAPHICA. SECTION B, STRUCTURAL CRYSTALLOGRAPHY AND CRYSTAL CHEMISTRY., vol. 29, no. 10, 1 October 1973 (1973-10-01), DK, pages 2163 - 2166, XP055716475, ISSN: 0567-7408, DOI: 10.1107/S056774087300628X

## Beschreibung

Die vorliegende Erfindung betrifft eine neue Kristallmodifikation von N-(Aminoiminomethyl)-2-aminoethansäure sowie ein Verfahren zur Herstellung dieser Kristallmodifikation.

N-(Aminoiminomethyl)-2-aminoethansäure (CAS-Nr. 352-97-6, Summenformel C₃H₇N₃O₂), auch als Guanidinoessigsäure, Guanidinoacetat, Glycocyamin, N-Amidinoglycin oder N-(Aminoiminomethyl)-glycin bekannt, ist eine Guanidinocarbonsäure mit vielfältigen Anwendungen, u.a. in der Synthese von chemischen Produkten, insbesondere Pharmazeutika (vgl. WO 2000/059528), zur direkten Anwendung als pharmazeutischer Wirkstoff bei Nierenerkrankungen (vgl. JP 60054320) oder neurodegenerativen Erkrankungen (vgl. CN 106361736), in der Herstellung von Polymeren (vgl. Du, Shuo et. al., Journal of Materials Science (2018), 53(1), 215-229), als Komplexbildner für Metalle (vgl. Lopes de Miranda et.al., Polyhedron (2003), 22(2), 225-233 bzw. Singh, Padmakshi et. al, Oriental Journal of Chemistry (2008), 24(1), 283-286) und als Zusatzstoff für die Ernährung von Tieren, insbesondere Säugetieren, Fischen, Vögeln (vgl. WO 2005/120246) und dem Menschen (vgl. WO 2008/092591, DE 102007053369).

N-(Aminoiminomethyl)-2-aminoethansäure lässt sich z.B. nach Strecker, M. (Jahresber. Fortschr. Chem. Verw. (1861), 530) aus Glycin durch Umsetzung mit Cyanamid herstellen. Alternativ kann N-(Aminoiminomethyl)-2-aminoethansäure z.B. durch Umsetzung von Glycin mit S-Methylisothioharnstoff-Jodid unter Verwendung von Kaliumhydroxid als Base hergestellt werden (vgl. US 2,654,779). Auch die Umsetzung von Chloressigsäure mit Ammoniak zu Glycinhydrochlorid und dessen weitere Umsetzung mit Cyanamid wurden beschrieben (vgl. US 2,620,354). Die Umsetzung von Cyanamid mit Glycin zu Glycocyamin bei einem pH-Wert von 9 bis 10 wurde in DE 964 590 beschrieben.

G.P. Jones und P. J. Pauling, Journal of Crystal and Molecular Structure, Vol. 9, No. 5, 1979, 273-279 beschreiben eine Kristall-und Molekularstruktur von Guanidinoessigsäure. S. Guha, Acta Cryst. (1973), B29, 2163-2166 beschreibt eine Kristall- und Molekularstruktur von Glycocyamin.

Bei den bekannten Verfahren fällt N-(Aminoiminomethyl)-2-aminoethansäure als feinkristallines Pulver an, welches einen erheblichen Staubanteil, d.h. einen erheblichen Anteil an Partikeln, die eine Korngröße kleiner 63 µm besitzen, aufweist.

Für die Handhabung von chemischen Produkten in fester Form ist es oftmals wünschenswert, dass diese in kristalliner, körniger, rieselfähiger, staubfreier Form ohne oder mit nur geringem Feinkornanteil vorliegen. Insbesondere für die Anwendung als Futtermittelzusatzstoff ist ein schlecht rieselfähiges, staubendes Pulver völlig ungeeignet.

Um diesem Sachverhalt zu begegnen, wurde beispielsweise vorgeschlagen, N-(Aminoiminomethyl)-2-aminoethansäure unter Zusatz von polymeren Bindemitteln (z.B. Methylcellulose) in Mengen von 0,05 bis 15 Gew.-% und Zusatz von Wasser zu Formlingen, Granulaten oder Extrudaten umzuformen (vgl. WO 2009/012960). Nachteilig an diesem Verfahren ist, dass ein Zusatz eines Fremdstoffs, nämlich eines Bindemittels, zwingend erforderlich ist, und dass in einem zusätzlichen Verfahrensschritt, unter Verwendung eines speziellen, technisch aufwendigen und teuren Apparats, wie beispielsweise einem Extruder, Granulator, Intensivmischer oder Pflugscharmischer, und nachfolgender Trocknung das Granulat bzw. die Formlinge hergestellt werden müssen.

Nachteilig am Verfahren gemäß obigem Stand der Technik ist zudem, dass Formlinge oder Granulate entweder einen hohen Bindemittelanteil und damit eine geringe Lösegeschwindigkeit aufweisen, oder aber bei einem geringen Bindemittelanteil sich zwar relativ schnell auflösen, zugleich aber eine geringe Festigkeit und hohe Abriebwerte aufweisen, so dass die Staubfreiheit nicht mehr gewährleistet werden kann.

Der Erfindung liegt daher die Aufgabe zugrunde, N-(Aminoiminomethyl)-2-aminoethansäure in Form von rieselfähigen, nicht staubenden Kristallaggregaten zur Verfügung zu stellen, welche die Nachteile des Standes der Technik nicht aufweisen, sondern einfach und mit verbreiteten Standardapparaten der chemischen Industrie herstellbar sind, und die zudem eine hohe Löslichkeit aufweisen. Zudem soll ein geeignetes Verfahren zur Herstellung dieser Kristallaggregate zur Verfügung gestellt werden.

Gelöst werden diese Aufgaben durch eine thermodynamisch metastabile Kristallmodifikation gemäß Anspruch 1 sowie ein Verfahren zur Herstellung derselben gemäß Anspruch 7. Bevorzugte Ausführungsformen der Erfindung sind in den Unteransprüchen angegeben, die wahlweise miteinander kombiniert werden können.

Das Auftreten von chemischen Stoffen in verschiedenen Kristallformen bzw. Kristallmodifikationen (Polymorphie) ist sowohl für die Herstellung und Anwendung der Stoffe als auch für die Entwicklung von Formulierungen von großer Bedeutung. So unterscheiden sich die verschiedenen Kristallmodifikationen einer chemischen Verbindung neben dem Aussehen (Kristallhabitus) auch in zahlreichen weiteren physikalischen oder physiko-chemischen Eigenschaften. Es ist bisher nicht möglich, das Auftreten und die Anzahl von Kristallmodifikationen einschließlich ihrer physikalischen oder physiko-chemischen Eigenschaften vorherzusagen. Vor allem die thermodynamische Stabilität und auch das unterschiedliche Verhalten nach Darreichung in lebenden Organismen lassen sich nicht im Voraus bestimmen.

Unter gegebenen Druck- und Temperaturbedingungen haben die verschiedenen polymorphen Kristallmodifikationen üblicherweise unterschiedliche Gitterenergien bzw. Standardbildungswärmen. Die Kristallform mit der niedrigsten Energie wird als stabile Form bezeichnet. Formen mit höherer energetischer Lage werden, sofern sie isoliert werden können, als metastabil (unter den gegebenen Druck- und Temperaturbedingungen) bezeichnet. Metastabile Polymorphe haben die Tendenz, sich in das stabile Polymorph umzuwandeln. Dies erfordert aufgrund der Metastabilität den Aufwand einer Aktivierungsenergie, z.B. durch Einwirkung von Wärme, mechanischer Energie oder durch Einfluss eines Lösemittels.

Zudem ist allgemein bekannt, dass die verschiedenen Modifikationen einer Substanz monotrop oder enantiotrop vorliegen können. Im Fall der monotropen Polymorphie kann eine Kristallform bzw. Kristallmodifikation über den gesamten Temperaturbereich bis zum Schmelzpunkt die thermodynamisch stabile Phase darstellen, wohingegen bei enantiotropen Systemen ein Umwandlungspunkt existiert, bei dem sich das Stabilitätsverhältnis umkehrt.

Überraschenderweise wurde eine bisher nicht bekannte, thermodynamisch metastabile Kristallmodifikation von N-(Aminoiminomethyl)-2-aminoethansäure gefunden. Somit ist gemäß einer ersten Ausführung der vorliegenden Erfindung eine thermodynamisch metastabile Kristallmodifikation von N-(Aminoiminomethyl)-2-aminoethansäure Gegenstand der vorliegenden Erfindung, die im Röntgen-Pulver-Diffraktogramm der Kristallmodifikation bei Verwendung von Cu-Kα₁-Strahlung die stärksten Reflexbanden bei 2Θ (2 Theta) = 20,2 ° und 23,3 ° und 23,8 ° und 25,3 ° bei einer Messgenauigkeit von +/- 0,2 ° zeigt.

Hierbei und im Folgenden bedeutet Cu-Kα₁-Strahlung eine Kupfer-K-alpha-1-Strahlung der Wellenlänge 1,5406 Å, wie sie üblicherweise in kristallographischen Untersuchungen herangezogen wird.

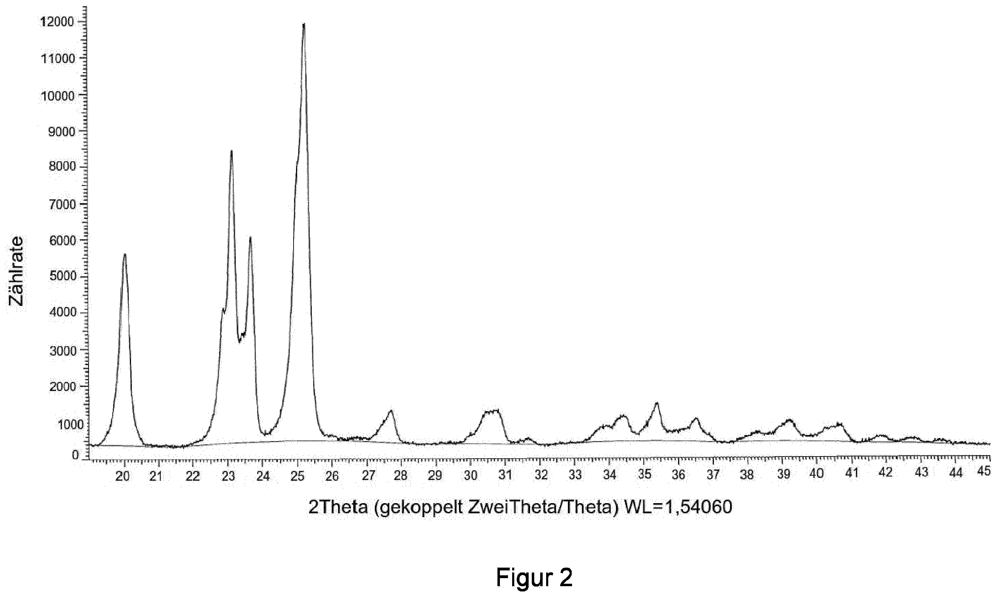

Bevorzugt weist die erfindungsgemäße thermodynamisch metastabile Kristallmodifikation von N-(Aminoiminomethyl)-2-aminoethansäure ein Röntgen-Pulver-Diffraktogramm bei Verwendung von Cu-Kα₁-Strahlung wie in Figur 2 dargestellt auf.

Bevorzugt ist eine thermodynamisch metastabile Kristallmodifikation von N-(Aminoiminomethyl)-2-aminoethansäure, die die orthorhombische Raumgruppe P2₁2₁2₁ mit Z = 8 mit den Gitterkonstanten a = 7,7685 Å, b = 7,7683 Å, c = 17,4261 Å bei einer Messgenauigkeit von +/- 0,001 Å aufweist. Die Einkristall-Messung erfolgte hierbei mit einer Mo-Kα-Strahlung der Wellenlänge 0,71073 Å bei 105 K (Kelvin). Das Elementarzellvolumen beträgt 1052 Å³ und die berechnete Röntgen-Kristalldichte 1,479 g/cm³ bei 105 Kelvin.

Weiter bevorzugt ist eine thermodynamisch metastabile Kristallmodifikation von N-(Aminoiminomethyl)-2-aminoethansäure, die in der orthorhombischen, polaren Raumgruppe P2₁2₁2₁ mit Z = 8, d.h. mit zwei kristallographisch unabhängigen Molekülen, kristallisiert und die eine pseudo-tetragonale Packung aufweist. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf. Die Einkristall-Messung erfolgte hierbei mit einer Mo-Kα-Strahlung der Wellenlänge 0,71073 Å. Das Elementarzellvolumen beträgt 1052 Å³ und die berechnete Röntgen-Kristalldichte 1,479 g/cm³ bei 105 Kelvin.

Gemäß der vorliegenden Erfindung bedeutet eine orthorhombische Raumgruppe eine Raumgruppe, deren Elementarzelle drei rechte Winkel (rechter Winkel = 90 °) aufweist und deren 3 Kristallachsen a, b und c unterschiedliche Längen aufweisen.

Gemäß einer bevorzugten Ausführung ist damit auch eine thermodynamisch metastabile Kristallmodifikation von N-(Aminoiminomethyl)-2-aminoethansäure Gegenstand der vorliegenden Erfindung, die im Röntgen-Pulver-Diffraktogramm der Kristallmodifikation bei Verwendung von Cu-Kα₁-Strahlung die stärksten Reflexbanden bei 2Θ = 20,2 ° und 23,3 ° und 23,8 ° und 25,3 ° bei einer Messgenauigkeit von +/- 0,2 ° zeigt, die in der orthorhombischen, polaren Raumgruppe P2₁2₁2₁ mit zwei kristallographisch unabhängigen Molekülen, d.h. Z = 8, kristallisiert und die eine pseudo-tetragonale Packung aufweist. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf.

Weiter bevorzugt weist die Kristallmodifikation ein Zellvolumen von 1052 Å³ und eine Röntgen-Kristalldichte von 1,479 g/cm³ bei 105 Kelvin auf.

Im Rahmen der vorliegenden Erfindung wurde gefunden, dass N-(Aminoiminomethyl)-2-aminoethansäure neben einer bereits bekannten, thermodynamisch stabilen Kristallmodifikation (im Folgenden auch Form A oder Kristallform A genannt) auch in einer thermodynamisch metastabilen Kristallmodifikation auftritt. Diese erfindungsgemäße thermodynamisch metastabile Kristallform wird im Folgenden auch Form B oder Kristallform B genannt.

Diese neue Kristallform B bildet bei geeigneten Kristallisationsbedingungen polygonale oder kugelige, radialstrahlige Aggregate aus nadeligen Teilkristalliten, die einen rundlichen Habitus und eine weitgehend einheitliche Aggregatgröße aufweisen. Damit stellen sie eine optimale Handhabung als Feststoff sicher, indem sie ein staubfreies, gut rieselfähiges Produkt ohne Verbackungsneigung ermöglichen. Die Kristallmodifikation B kann als staubarm eingestuft werden, da der Anteil an Kristallen mit einer Korngröße von < 63 µm (Mesh-Größe) unter 10 %, vorzugsweise unter 5 %, liegt. Durch ihren Aufbau aus feinen, nadeligen Teilkristalliten stellt dieser Habitus der neuen Kristallform B von N-(Aminoiminomethyl)-2-aminoethansäure zudem eine höhere Lösegeschwindigkeit sicher. Zusätzlich und völlig unerwartet bietet N-(Aminoiminomethyl)-2-aminoethansäure der Kristallform B außerdem eine höhere absolute Löslichkeit in Wasser-haltigen Medien.

Die erfindungsgemäße metastabile Kristallmodifikation von N-(Aminoiminomethyl)-2-aminoethansäure liegt bevorzugt in reiner Form vor. Vorzugsweise weisen mindestens 50 Gew.-%, mehr bevorzugt mindestens 75 Gew.-%, noch mehr bevorzugt mindestens 90 Gew.-%, insbesondere mindestens 95 Gew.-% und am meisten bevorzugt mindestens 99 Gew.-% der N-(Aminoiminomethyl)-2-aminoethansäure in einer Zusammensetzung eine Kristallmodifikation im Röntgen-Pulver-Diffraktogramm bei Verwendung von Cu-Kα₁-Strahlung mit stärksten Reflexbanden bei 2Θ = 20,2 ° und 23,3 ° und 23,8 ° und 25,3 ° bei einer Messgenauigkeit von +/- 0,2 ° auf.

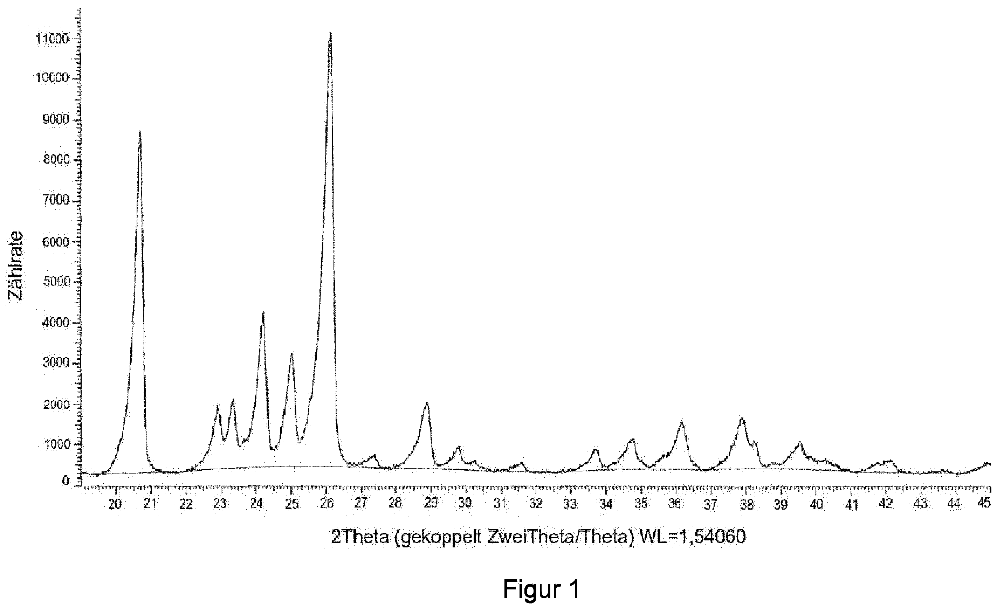

Wird N-(Aminoiminomethyl)-2-aminoethansäure nach einem der bekannten Verfahren, insbesondere aus wasserhaltigen Reaktionsmischungen, hergestellt, so fällt die Verbindung in der wohlbekannten Kristallform A an. Ein und dieselbe Kristallstruktur wurde von drei Autorengruppen beschrieben: von Sankarananda Guha, Acta Cryst. B29 (1973), 2163 bzw. von Par J. Berthou et. al., Acta Cryst B32 (1976), 1529 und von Wei Wang et. al, Tetrahedron Letters 56 (2015), 2684. In allen drei Arbeiten wird N-(Aminoiminomethyl)-2-aminoethansäure (hier Form A genannt) beschrieben als monokline Struktur der Raumgruppe P2₁/n mit Z = 4 und den angenäherten Gitterkonstanten a = 4,95 Å, b = 6,00 Å, c = 17,2 Å, β = 94,5 °, mit einem Zellvolumen von ca. 510 Å³, wobei bei Berthou et. al. die publizierte Raumgruppe P2,/c über eine Koordinatentransformation zur Raumgruppe P2₁/n überführt wurde. Die experimentelle Kristalldichte von N-(Aminoiminomethyl)-2-aminoethansäure der Form A beträgt ca. 1,50 g/cm³. Das charakteristische Pulverdiffraktogramm von N-(Aminoiminomethyl)-2-aminoethansäure in Form A ist in Figur 1 gezeigt. Unter Verwendung von Cu-Kα₁-Strahlung (Kupfer-K-alpha-1-Strahlung) ist für Form A insbesondere die Bandenlage 2Θ (2Theta) = 20,6 ° und 26,0 ° charakteristisch. Das Pulverdiffraktogramm stimmt mit dem aus den publizierten Einkristallstrukturen berechneten Beugungsmuster überein.

Wird N-(Aminoiminomethyl)-2-aminoethansäure aus üblichen Lösemitteln, wie beispielsweise Wasser, Methanol, Ethanol, Isopropanol oder Mischungen aus Methanol, Ethanol, Ethandiol, oder Acetonitril mit Wasser, umkristallisiert oder darin hergestellt, so fällt N-(Aminoimino-methyl)-2-aminoethansäure ausschließlich in Kristallform A an, wie durch Versuche gezeigt werden konnte.

Völlig überraschend wurde gefunden, dass N-(Aminoiminomethyl)-2-aminoethansäure aus einer Calciumchlorid-haltigen Lösung, insbesondere einer wässrigen Calciumchloridlösung mit einem Calciumchlorid-Anteil von 5 bis 50 Gew.-%, ganz besonders mit einem Calciumchlorid-Anteil von 10 bis 40 Gew.-%, in einer neuen, bislang unbekannten Kristallform kristallisiert.

N-(Aminoiminomethyl)-2-aminoethansäure der Form B wird charakterisiert durch ihr Pulverdiffraktogramm mit Cu-Kα₁-Strahlung (siehe Figur 2), wobei die Banden bei 2Θ (2 Theta) = 20,2 ° und 25,3 ° sowie ein schwächerer Doppelreflex bei 2Θ (2Theta) = 23,3 °/ 23,8 ° charakteristisch sind. Eine Einkristall-Röntgenstrukturanalyse ergab für N-(Aminoiminomethyl)-2-aminoethansäure der Form B die orthorhombische, polare Raumgruppe P2₁2₁2₁ mit zwei kristallographisch unabhängigen Molekülen, d.h. Z = 8. Die Packung der Moleküle weist eine pseudo-tetragonale Symmetrie auf. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf. Die Einkristall-Messung erfolgte hierbei mit einer Mo-Kα-Strahlung der Wellenlänge 0,71073 Å. Das Elementarzellvolumen beträgt 1052 Å³ und die berechnete Röntgen-Kristalldichte 1,479 g/cm³ bei 105 Kelvin.

Gemäß einer weiteren Ausführung ist damit auch eine thermodynamisch metastabile Kristallmodifikation von N-(Aminoiminomethyl)-2-aminoethansäure Gegenstand der vorliegenden Erfindung, die in einer orthorhombischen Raumgruppe P2₁2₁2₁, insbesondere orthorhombischen, polaren Raumgruppe P2₁2₁2₁, mit Z = 8 kristallisiert und die insbesondere eine pseudo-tetragonale Packung aufweist. Die Elementarzelle weist bei 105 Kelvin die Gitterkonstanten a = 7,7685 Å, b = 7,7683 Å, c = 17,4261 Å bei einer Messgenauigkeit von +/- 0,001 Å auf.

Die experimentelle Kristalldichte von N-(Aminoiminomethyl)-2-aminoethansäure der Form B beträgt 1,41 g/cm³ +/- 0,03 g/cm³ bei 20 °C. Somit liegt damit die experimentelle Kristalldichte der Form B deutlich unter derjenigen von Kristallform A, die 1,50 g/cm³ +/- 0,03 g/cm³ bei 20 °C beträgt. Dieser Unterschied in der Kristalldichte deutet auf eine thermodynamische Instabilität von Form B gegenüber Form A hin.

Somit ist bevorzugt auch eine thermodynamisch metastabile Kristallmodifikation Gegenstand der Erfindung, die eine experimentelle Kristalldichte von 1,41 g/cm³ +/- 0,03 g/cm³ bei 20 °C aufweist.

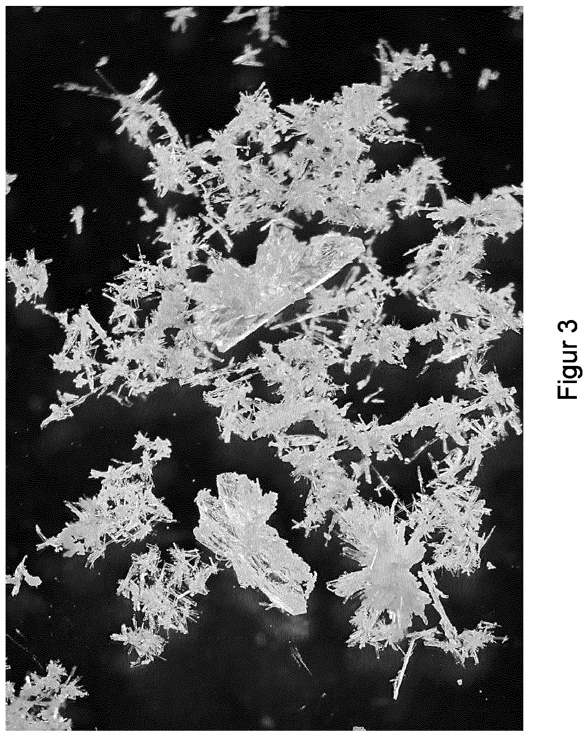

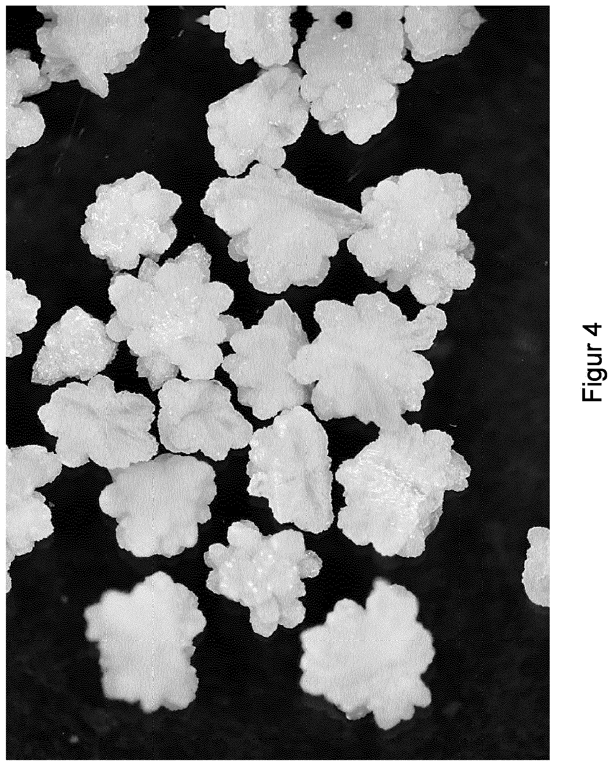

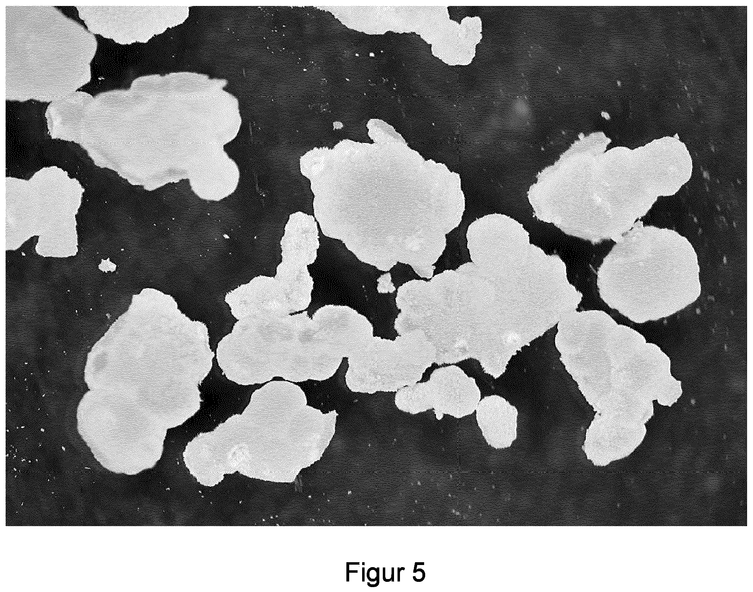

Kristallform B von N-(Aminoiminomethyl)-2-aminoethansäure liegt vorzugsweise in Form kugeliger oder polygonaler, radialstrahliger Aggregate mit äußerem rundlichem Habitus vor. Die Einzelkristalle stellen feinste Nadeln dar, aus denen die kugeligen Aggregate aufgebaut sind. Dies hat den überraschenden Vorteil, dass mittels Form B eine physikalische Form von N-(Aminoiminomethyl)-2-aminoethansäure bereitgestellt werden kann, die kugelige oder polygonale, körnige, abriebfeste Aggregate umfasst, mit einer weitgehend einheitlichen Aggregatgröße, einer hervorragenden Rieselfähigkeit und weitgehender Staubfreiheit. Typische Kristallaggregate von N-(Aminoiminomethyl)-2-aminoethansäure der Form B sind in Figur 4 und Figur 5 gezeigt. Zum Vergleich wird übliche, dem Stand der Technik entsprechende N-(Aminoiminomethyl)-2-aminoethansäure der Form A, die den Habitus verfilzter, feiner Kristallnadeln aufweist, in Figur 3 gezeigt.

N-(Aminoiminomethyl)-2-aminoethansäure Form A und Form B unterscheiden sich zudem im Infrarotspektrum. Charakteristisch für Form A sind stärkere Banden bei 1005,9, 940,3 und 816,8 cm⁻¹, charakteristisch für Form B sind stärkere Banden bei 1148,0, 997,7 und eine nur schwache Bande bei 815 cm⁻¹.

Die beiden Kristallformen zeigen unterschiedliche Schmelz- bzw. Zersetzungspunkte:
N-(Aminoiminomethyl)-2-aminoethansäure Form A: DSC onset 280,5 °C, peak 286,3 °C, Schmelzwärme 887 +/- 1 J/g.
N-(Aminoiminomethyl)-2-aminoethansäure Form B: DSC onset 272,5 °C, peak 280,4 °C, Schmelzwärme 860 +/- 1 J/g.

Somit ist bevorzugt auch eine thermodynamisch metastabile Kristallmodifikation Gegenstand der Erfindung, die endotherme Schmelzwärme im Bereich von 850 bis 870 J/g aufweist.

Weiterhin bevorzugt ist somit auch eine thermodynamisch metastabile Kristallmodifikation Gegenstand der Erfindung, die einen Zersetzungspunkt im Bereich von 270 bis 275 °C aufweist.

Diese Daten zeigen eindrucksvoll, dass N-(Aminoiminomethyl)-2-aminoethansäure Form B eine thermodynamisch metastabile Kristallmodifikation ist, die im Vergleich zur Form A die thermodynamisch instabilere Form darstellt, wobei der Energieunterschied zwischen beiden Formen ca. 27 J/g beträgt und wobei der Anfangspunkt der Schmelzbereiche (onset) einen Unterschied von 8 K aufweist.

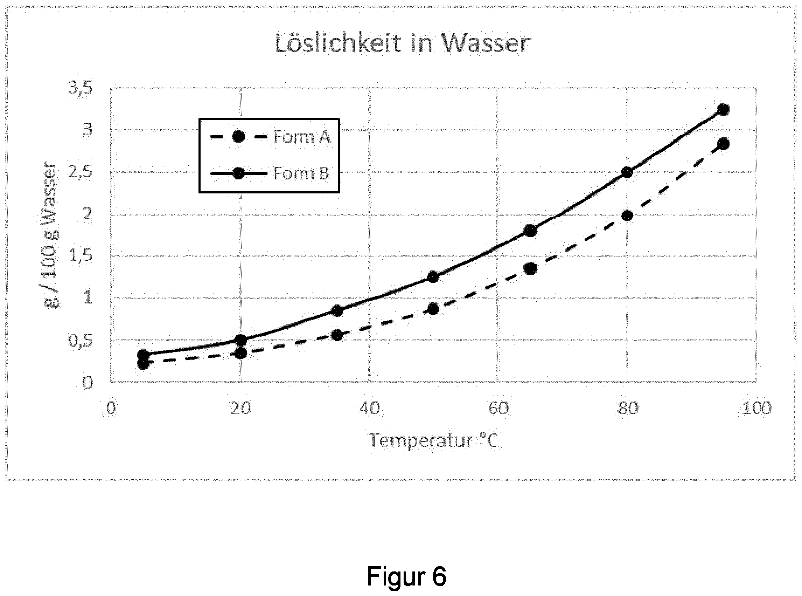

In weitergehenden Untersuchungen hat sich gezeigt, dass N-(Aminoiminomethyl)-2-aminoethansäure Form B eine um ca. 20 % höhere Wasserlöslichkeit als N-(Aminoiminomethyl)-2-aminoethansäure Form A aufweist, und dass dieser Sachverhalt im Temperaturbereich zwischen 5 und 95 °C zutrifft (vgl. Figur 6). Dieser Effekt ist vollkommen unvorhersehbar.

Es wurde gefunden, dass die erfindungsgemäße metastabile Kristallmodifikation Form B als Feststoff bis zu ihrem Schmelzpunkt stabil vorliegt. Eine Feststoffumwandlung von Form B in die Form A oder eine reversible Feststoffumwandlung Form A / Form B kann nicht beobachtet werden. Somit liegt mit Form B ein Beispiel für eine monotrope Polymorphie vor.

Zusammenfassend ist hier hervorzuheben, dass N-(Aminoiminomethyl)-2-aminoethansäure in der Kristallmodifikation B, insbesondere hergestellt durch Kristallisation von N-(Amino-iminomethyl)-2-aminoethansäure aus einer Calciumchlorid enthaltenden, bevorzugt wässrigen oder wasserhaltigen Lösung, überraschenderweise vorteilhafte und gewöhnlich entgegengesetzte Eigenschaften, wie z.B. ein grobes, rieselfähiges Korn und zugleich eine hohe Lösegeschwindigkeit, die Bildung von Kristallaggregaten ohne Zusatz eines Bindemittels, kombiniert, und eine erhöhte absolute Löslichkeit bei gegebener Temperatur, trotz identischer chemischer Zusammensetzung bereitstellt.

Diese neue Kristallmodifikation ist aufgrund ihrer hervorragenden Eigenschaften geeignet, als Futtermittelzusatz für Tiere eingesetzt zu werden. Somit ist auch ein Futtermittelzusatz umfassend die hierin beschriebene thermodynamisch metastabile Kristallmodifikation von N-(Aminoiminomethyl)-2-amino-ethansäure Gegenstand der vorliegenden Erfindung.

Insbesondere ist somit auch ein Futtermittelzusatz umfassend eine thermodynamisch metastabile Kristallmodifikation von N-(Aminoiminomethyl)-2-aminoethansäure Gegenstand der vorliegenden Erfindung, dessen Röntgen-Pulver-Diffraktogramm bei Verwendung von Cu-Kα₁-Strahlung die stärksten Reflexbanden bei 2Θ = 20,2 ° und 23,3 ° und 23,8 ° und 25,3 ° bei einer Messgenauigkeit von +/-0,2 ° zeigt.

Ganz besonders bevorzugt ist ein Futtermittelzusatz umfassend eine thermodynamisch metastabile Kristallmodifikation von N-(Aminoiminomethyl)-2-aminoethansäure, der keine Bindemittel umfasst oder der frei von Bindemitteln ist, die üblicherweise zur Granulation eingesetzt werden.

Derartige Futtermittelzusätze können als Premixe formuliert werden. Somit ist zudem auch die Verwendung der hierin beschriebenen thermodynamisch metastabilen Kristallmodifikation von N-(Aminoiminomethyl)-2-aminoethansäure zur Herstellung eines Futtermittelzusatzes Gegenstand der Erfindung.

Überraschenderweise wurde gefunden, dass N-(Aminoiminomethyl)-2-aminoethansäure in unterschiedlichen Kristallmodifikationen vorliegenden kann. Die erfindungsgemäße, thermodynamisch metastabile Kristallform B von N-(Aminoiminomethyl)-2-aminoethansäure kann hergestellt werden, indem N-(Aminoiminomethyl)-2-aminoethansäure in Gegenwart von Calciumchlorid zur Kristallisation gebracht wird.

Der Anteil an Calciumchlorid (bezogen auf das wasserfreie Salz) beträgt vorzugsweise mindesten 5 bis maximal 50 Gew-%, besonders bevorzugt 10 bis 40 Gew-%. Der Anteil an Calciumchlorid bezieht sich dabei auf das Gesamtgewicht der Lösung, die zur Kristallisation der N-(Aminoiminomethyl)-2-aminoethansäure eingesetzt wird.

Weiterhin bevorzugt kann das Calciumchlorid (bezogen auf das wasserfreie Salz) in einer Menge von mindestens 5 Gew.-% und höchstens 50 Gew.-% (bezogen auf das Gesamtgewicht der Lösung) eingesetzt werden. Bevorzugt kann das Calciumchlorid in einer Menge von mindestens 7 Gew.-%, weiter bevorzugt mindestens 10 Gew.-%, besonders bevorzugt mindestens 15 Gew.-% und ganz besonders bevorzugt mindestens 20 Gew.-%, wobei weiterhin bevorzugt höchstens 50 Gew.-% (jeweils bezogen auf das Gesamtgewicht der Lösung) eingesetzt werden. Gleichzeitig kann das Calciumchlorid in einer Menge von höchstens 50 Gew.-%, weiter bevorzugt von höchstens 45 Gew.-% und ganz besonders bevorzugt höchstens 40 Gew.-% (jeweils bezogen auf das Gesamtgewicht der Lösung) eingesetzt werden.

Somit ist auch ein Verfahren zur Herstellung dieser thermodynamisch metastabilen Kristallmodifikation von N-(Aminoiminomethyl)-2-aminoethansäure, wobei die Kristallmodifikation im Röntgen-Pulver-Diffraktogramm der Kristallmodifikation bei Verwendung von Cu-Kα₁-Strahlung die stärksten Reflexbanden bei 2Θ = 20,2 ° und 23,3 ° und 23,8 ° und 25,3 ° bei einer Messgenauigkeit von +/- 0,2 ° zeigt, Gegenstand der vorliegenden Erfindung, indem N-(Aminoiminomethyl)-2-aminoethansäure aus einer Lösung enthaltend 5 bis 50 Gew.-% Calciumchlorid, vorzugsweise 5 bis 40 Gew.-% Calciumchlorid, vorzugsweise 10 bis 40 Gew.-% Calciumchlorid, kristallisiert wird.

Das Verfahren kann durchgeführt werden:
a) durch Umkristallisation von N-(Aminoiminomethyl)-2-aminoethansäure einer beliebigen Kristallstruktur aus einem Calciumchlorid enthaltenden Lösemittel, oder
b) durch Synthese von N-(Aminoiminomethyl)-2-aminoethansäure, vorzugsweise durch Umsetzung von Glycin mit Cyanamid in einem Calciumchlorid enthaltenden Lösemittel.

Bevorzugt kann das Verfahren durchgeführt werden, indem die N-(Aminoiminomethyl)-2-aminoethansäure in einem Temperaturbereich von -40 bis 100 °C kristallisiert wird.

Besonders bevorzugt ist ein Verfahren, indem N-(Aminoiminomethyl)-2-aminoethansäure mit einer Abkühlrate im Bereich von 0,01 bis 5 K/min in einem Temperaturbereich von -40 bis 100 °C kristallisiert wird.

Als Lösemittel können weiter bevorzugt Wasser, Alkohole, Ester, Nitrile, Ketone oder Mischungen dieser Lösemittel eingesetzt werden. Bevorzugt sind wässrige oder wasserhaltige Lösemittelgemische, ganz besonders bevorzugt ist Wasser.

Überraschenderweise wurde zudem gefunden, dass die die Anwesenheit von Calciumchlorid die Löslichkeit von N-(Aminoiminomethyl)-2-aminoethansäure sehr deutlich erhöht. Dies kann unter anderem auch vorteilhaft sein, wenn höher konzentrierte Lösungen oder flüssige Formulierungen von N-(Aminoiminomethyl)-2-aminoethansäure gewünscht werden.

Im genannten Calciumchlorid enthaltenden Lösemittel oder Lösemittelgemisch wird N-(Aminoiminomethyl)-2-aminoethansäure entweder gemäß a) bis zur Sättigung gelöst oder gemäß b) aus Glycin und Cyanamid durch eine Synthesereaktion erzeugt, so dass mit fortschreitender Reaktion der Sättigungspunkt erreicht wird. Gemäß dem Verfahren der Erfindung erfolgt die Kristallkeimbildung und Kristallisation vorzugsweise in Form B, wobei die Anwesenheit von Calciumchlorid als erfindungswesentlich anzusehen ist.

N-(Aminoiminomethyl)-2-aminoethansäure der Form B kann somit bevorzugt hergestellt werden, durch
a1) Abkühlkristallisation einer bei erhöhter Temperatur gesättigten Lösung die Übersättigung erreicht wird, oder
a2) Verdampfen eines Teils des Lösemittels bei im Wesentlichen konstanter Temperatur die Übersättigung erreicht wird,
   oder
b) Reaktion unter Bildung von N-(Aminoiminomethyl)-2-aminoethansäure wobei der Sättigungspunkt der Löslichkeit überschritten wird,
so dass N-(Aminoiminomethyl)-2-aminoethansäure Form B kristallisiert.

In allen Fällen werden vorzugsweise gewöhnliche Rührreaktoren eingesetzt. Der Einsatz aufwendiger verfahrenstechnischer Apparate ist nicht erforderlich.

Der bevorzugte Temperaturbereich für Verfahren gemäß a1), a2) oder b) beträgt -40 bis 110 °C, besonders bevorzugt -20 bis 100 °C.

Für Verfahren gemäß a1) beträgt die Lösetemperatur vorzugsweise 40 bis 110 °C, die Kristallisationstemperatur vorzugsweise -40 bis 40 °C.

Verfahren gemäß a2) werden vorzugsweise im Temperaturbereich 30 bis 100 °C durchgeführt.

Verfahren gemäß b) erfolgen vorzugsweise im Temperaturbereich zwischen 60 und 100 °C.

Nach vollständiger Kristallisation der gewünschten N-(Aminoiminomethyl)-2-aminoethansäure Form B wird das auskristallisierte Produkt vorzugsweise durch Filtration, z.B. mittels Zentrifuge, Druckfilternutsche, Bandfilter oder Filterpresse abfiltriert. Zur Entfernung überschüssigen Calciumchlorids wird vorzugsweise mit dem oben genannten Lösemittel oder Lösemittelgemisch nachgewaschen. Vorzugsweise wird mit Wasser gewaschen, wobei die Temperatur des Waschwassers bevorzugt 0 bis 50 °C beträgt.

Selbstverständlich ist es zur Verbesserung der Wirtschaftlichkeit des Verfahrens möglich, die aus der Abtrennung der N-(Aminoiminomethyl)-2-aminoethansäure der Kristallform B erhaltene Mutterlauge in den Prozess zurückzuführen, ggf. unter Einstellung der Konzentration von Calciumchlorid, z.B. durch Eindampfung. Nach Trocknung, vorzugsweise im Temperaturbereich 40 bis 100 °C, liefert das erfindungsgemäße Verfahren ein trockenes, rieselfähiges, körniges Produkt bestehend aus radialstrahligen, polygonalen oder rundlichen Aggregaten. Die Kristallaggregate haben eine äußere Abmessung von 150 bis 3000 µm, vorzugsweise 300 bis 1500 µm und einen Staubanteil (d.h. Partikelanteil kleiner 63 µm) von weniger als 5 Gew.-%, Partikelgrößen jeweils Mesh-Größe).

Die so hergestellte N-(Aminoiminomethyl)-2-aminoethansäure Form B hat eine hohe Reinheit, typischerweise > 99,0 %, ist gut handhabbar und zeigt kaum mechanischen Abrieb. Aufgrund dieser Eigenschaften ist die erfindungsgemäße Kristallform B von N-(Aminoiminomethyl)-2-aminoethansäure besonders geeignet für die obengenannten Einsatzzwecke, insbesondere als Zusatzstoff zur Ernährung bzw. als pharmazeutischer Wirkstoff.

Ein weiterer Gegenstand der vorliegenden Erfindung ist deshalb die Verwendung der themodynamisch metastabilen Kristallmodifikation von N-(Aminoiminomethyl)-2-aminoethansäure wie hierin beschrieben zur Herstellung eines Futtermittelzusatzes sowie ein Futtermittelzusatz umfassend eine themodynamisch metastabile Kristallmodifikation von N-(Aminoiminomethyl)-2-aminoethansäure wie hierin beschrieben. Der Futtermittelzusatz ist insbesondere zur Fütterung von Geflügel geeignet

Die nachfolgenden Beispiele sollen das Wesen der Erfindung näher erläutern.

In den Abbildungen wird gezeigt:
- Figur 1:: Röntgen-Pulver-Diffraktogramm von N-(Aminoiminomethyl)-2-aminoethansäure der Form A aus Beispiel 1
- Figur 2:: Röntgen-Pulver-Diffraktogramm von N-(Aminoiminomethyl)-2-aminoethansäure der Form B aus Beispiel 2
- Figur 3:: Mikrophotographie von N-(Aminoiminomethyl)-2-aminoethansäure der Form A, hergestellt nach Beispiel 1 (Bildbreite 8 mm)
- Figur 4:: Mikrophotographie von polygonalen Aggregaten von N-(Aminoiminomethyl)-2-aminoethansäure der Form B, hergestellt durch Umkristallisation aus einer 30 %-igen wässrigen Calciumchloridlösung gemäß Beispiel 2 (Bildbreite 8 mm)
- Figur 5:: Mikrophotographie von kugeligen Aggregaten von N-(Aminoiminomethyl)-2-aminoethansäure der Form B, hergestellt durch Umkristallisation aus einer 15 %-igen wässrigen Calciumchloridlösung gemäß Beispiel 3 (Bildbreite 8 mm)
- Figur 6:: Löslichkeitskurve von N-(Aminoiminomethyl)-2-aminoethansäure der Form A bzw. Form B in Wasser
- Figur 7:: Abbildung der beiden kristallographisch unabhängigen Moleküle N-(Aminoiminomethyl)-2-aminoethansäure aus der Einkristall-Röntgenstrukturanalyse
- Figur 8:: Abbildung der Packung der Moleküle von N-(Aminoiminomethyl)-2-aminoethansäure im Kristallverband. Die Blickrichtung ist entlang der a-Achse. Deutlich sind voneinander unabhängige, senkrecht zueinander angeordnete, über H-Brücken gebundene Molekülketten parallel der a- und der b-Achse zu sehen. Diese Ketten sind entlang der c-Achse gestapelt.

### Beispiele

### Röntgen-Pulver-diffraktometrische Messung

Im Umfang der vorliegenden Beispiele wurden Röntgen-Pulver-diffraktometrische Messungen unter Verwendung eines Pulver-Diffraktometers Bruker D2 Phaser mit Theta/2Theta-Geometrie, einem LYNXEYE-Detektor, Cu-Kα₁-Strahlung der Wellenlänge 1,5406 Å mit einer Beschleunigungsspannung von 30 kV und einem Anodenstrom von 10 mA, einem Nickelfilter und einer Schrittweite von 0,02 ° durchgeführt. Die zur Untersuchung stehenden Proben wurden im Achatmörser vermahlen und gemäß Herstellerangaben auf den Probenteller gedrückt und die Oberfläche geglättet.

### Einkristall-Röntgenstrukturanalyse

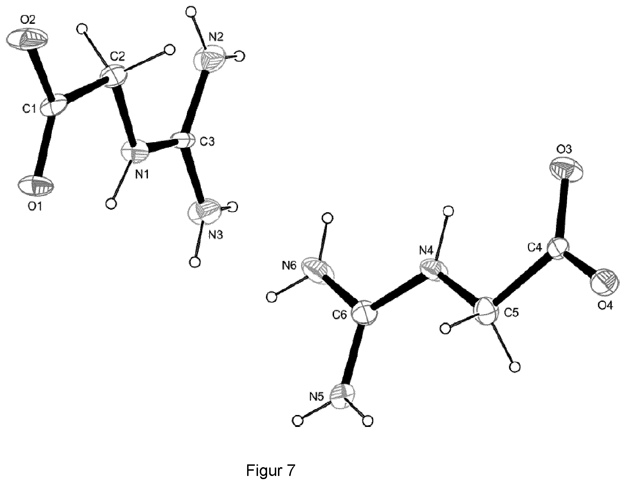

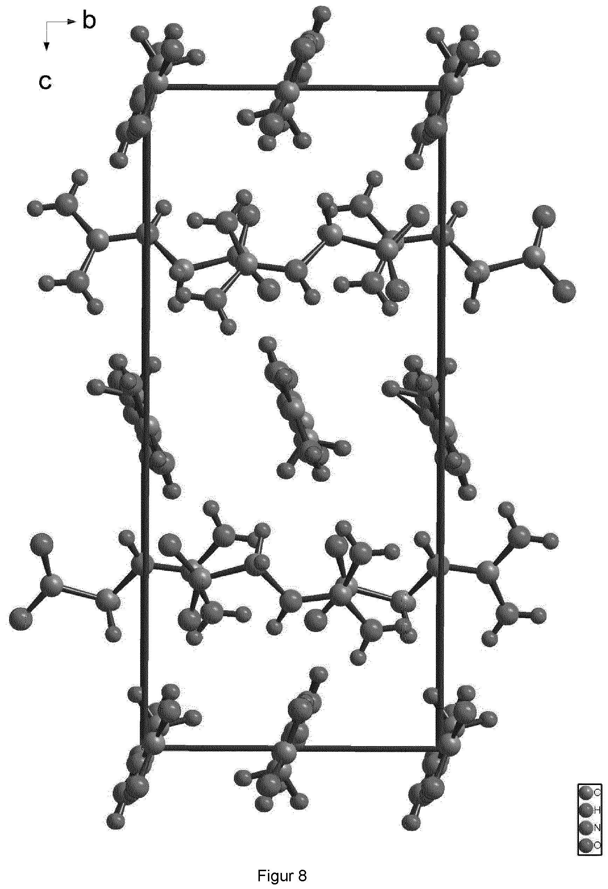

Ein geeigneter Kristall wurde durch Verdunsten einer wässrigen Lösung von N-(Aminoiminomethyl)-2-aminoethansäure in Gegenwart von Calciumchlorid hergestellt. Die Einkristallmessung erfolgte bei 105 Kelvin an einem Kristall der Dimension 0,02 * 0,02 * 0,09 mm unter Verwendung monochromatischer Mo-Kα (Molybdän-K-alpha)-Strahlung der Wellenlänge 0,71073 Å unter Einsatz eines Zweikreis-Diffraktometers Bruker D8 Venture TXS. Die Verfeinerung der Röntgenkristalldaten unter Verwendung von 2072 unabhängigen Reflexen erfolgte nach der Methode der kleinsten Fehlerquadrate bis zu einem R-Wert (F_{obs}) von 0,0381. Die Position der NH- und OH-Wasserstoffatome wurde verfeinert, die der CH-Wasserstoffatome an der berechneten Position fixiert. Das Ergebnis der Röntgen-Einkristallstrukturanalyse ist in Figur 7 und 8 veranschaulicht. Ein aus der Einkristallstrukturanalyse rückgerechnetes Pulverdiffraktogramm stimmte exakt mit dem gemessenen Pulverdiffraktogramm gemäß Figur 2 überein.

### Beispiel 1 (Vergleich) - Umkristallisation von N-(Aminoiminomethyl)-2-aminoethansäure aus Wasser

400 g Wasser wurden bei 80 °C vorgelegt und löffelweise insgesamt 11,66 g N-(Aminoiminomethyl)-2-aminoethansäure mit einem Gehalt von 99,0 %, vorliegend in Kristallform A, darin gelöst, wobei mit der letzten Portion die Löslichkeitsgrenze überschritten wurde. Dann wurde bei 80 °C abfiltriert, das Filtrat mit weiteren 100 g Wasser versetzt und auf 80 °C erhitzt. Es bildete sich eine knapp gesättigte, klare Lösung. Durch langsame Abkühlung auf 20 °C innerhalb von 4 Stunden wurde N-(Aminoiminomethyl)-2-aminoethansäure kristallisiert. Die ausgefallenen Kristalle wurden abfiltriert und bei 60 °C im Vakuum getrocknet. Es wurden 6,51 g N-(Aminoiminomethyl)-2-aminoethansäure mit einem Gehalt von 99,1 % erhalten.

Das erhaltene Produkt liegt in Form fein-nadeliger Kristalle vor. Die fein-nadeligen Kristalle wurden mikroskopisch untersucht (siehe Figur 3). Eine Röntgen-Pulver-diffraktometrische Messung ergab das mit Figur 1 gezeigte Pulverdiffraktogramm, welches die altbekannte Kristallform A anzeigt.

### Beispiel 2 (erfindungsgemäß) - Umkristallisation von N-(Aminoiminomethyl)-2-aminoethansäure aus einer 30 %-igen Calciumchloridlösung

Aus 150 g wasserfreiem Calciumchlorid und 350 g Wasser wurde eine 30 %-ige Lösung hergestellt. In 400 g dieser Lösung wurde bei 80 °C löffelweise N-(Aminoiminomethyl)-2-aminoethansäure derselben Zusammensetzung wie in Beispiel 1 (d.h. 99,0 % Gehalt, Kristallform A) zugesetzt. Erst bei einer zugegebenen Menge von 74,28 g war die Löslichkeitsgrenze überschritten. Der geringe Feststoffanteil wurde bei 80 °C abfiltriert, nicht gewaschen, das Filtrat mit den restlichen 100 g der 30 %-igen Lösung von Calciumchlorid versetzt und bei 80 °C für 1 Stunde gerührt. Es wurde eine klare, farblose Lösung erhalten. Durch langsame Abkühlung auf 20 °C innerhalb von 4 Stunden wurde N-(Aminoiminomethyl)-2-aminoethansäure kristallisiert. Die ausgefallenen Kristallaggregate wurden abfiltriert, 3 mal mit Wasser von 20 °C gewaschen und bei 60 °C getrocknet. Es wurden 46,42 g N-(Aminoiminomethyl)-2-aminoethansäure mit einem Gehalt von 99,2 % erhalten. Die erhaltene Menge ist somit über 7 mal größer als in Beispiel 1, was auf die durch Calciumchlorid stark erhöhte Löslichkeit von N-(Aminoiminomethyl)-2-aminoethansäure zurückzuführen ist.

Ein analog aufgenommenes Pulverdiffraktogramm (siehe Figur 2) zeigte die bislang unbekannte Kristallform B an. Die polygonalen, rundlichen Kristallaggregate wurden mikroskopisch untersucht (siehe Figur 4).

### Beispiel 3 (erfindungsgemäß) - Umkristallisation von N-(Aminoiminomethyl)-2-aminoethansäure aus einer 15 %-igen Calciumchloridlösung

Beispiel 2 wurde analog wiederholt mit 500 g einer 15 %-igen Calciumchloridlösung, hergestellt aus 75 g wasserfreiem Calciumchlorid und 425 g Wasser. In 400 g dieses Lösemittelgemisches wurde bei 80 °C die Sättigungsgrenze mit 42,7 g N-(Aminoiminomethyl)-2-aminoethansäure erreicht. Nach Zugabe der restlichen 100 g Lösemittel, Kristallisation der anfangs klaren Lösung, Filtration, Waschen und Trocknen wurden 27,2 g N-(Aminoiminomethyl)-2-aminoethansäure mit einem Gehalt von 99,2 % erhalten.

Das Pulverdiffraktogramm der kugeligen Kristallaggregate zeigte die alleinige Anwesenheit von Form B an. Die kugeligen, radialstrahligen Aggregate wurden mikroskopisch untersucht (siehe Figur 5).

### Beispiel 3a (erfindungsgemäß) - Umkristallisation von N-(Aminoiminomethyl)-2-aminoethansäure aus einer 10 %-igen Calciumchloridlösung

Beispiel 2 wurde analog wiederholt mit 500 g einer 10 %-igen Calciumchloridlösung, hergestellt aus 50 g wasserfreiem Calciumchlorid und 450 g Wasser. In 400 g dieses Lösemittelgemisches wurde bei 80 °C die Sättigungsgrenze mit 29,4 g N-(Aminoiminomethyl)-2-aminoethansäure erreicht. Nach Zugabe der restlichen 100 g Lösemittel, Kristallisation der anfangs klaren Lösung, Filtration, Waschen und Trocknen wurden 23,5 g N-(Aminoiminomethyl)-2-aminoethansäure mit einem Gehalt von 99,3 % erhalten.

Das Pulverdiffraktogramm zeigte die Anwesenheit einer Mischung von Kristallform A und Kristallform B an. Das Verhältnis beider Polymorphe betrug ca. 1:1.

### Beispiel 3b (Vergleich) - Umkristallisation von N-(Aminoiminomethyl)-2-aminoethansäure aus einer 1 %-igen Calciumchloridlösung

Beispiel 2 wurde analog wiederholt mit 500 g einer 1 %-igen Calciumchloridlösung, hergestellt aus 5 g wasserfreiem Calciumchlorid und 495 g Wasser. In 400 g dieses Lösemittelgemisches wurde bei 80 °C die Sättigungsgrenze mit 13,4 g N-(Aminoiminomethyl)-2-aminoethansäure erreicht. Nach Zugabe der restlichen 100 g Lösemittel, Kristallisation der anfangs klaren Lösung, Filtration, Waschen und Trocknen wurden 11,0 g N-(Aminoiminomethyl)-2-aminoethansäure mit einem Gehalt von 99,4 % erhalten.

Das Pulverdiffraktogramm der fein-nadeligen Kristallaggregate zeigte die alleinige Anwesenheit von Form A an.

Abhängig von der Calciumchloridkonzentration lässt sich somit das Entstehen von Form A oder Form B beeinflussen. Die Löslichkeit (d.h. Sättigungsgrenze) von N-(Aminoiminomethyl)-2-aminoethansäure steigt mit der CalciumchloridKonzentration stark an.

### Beispiel 4 (Vergleich) - Umkristallisation von N-(Aminoiminomethyl)-2-aminoethansäure aus einer 50 %-igen Lösung von Magnesiumchlorid-Hexahydrat

Beispiel 2 wurde analog wiederholt mit 500 g einer Lösung hergestellt aus 250 g Magnesiumchlorid-Hexahydrat und 250 g Wasser. In 400 g des Lösemittelgemisches wurde bei 80 °C die Sättigungsgrenze mit 76,6 g N-(Aminoiminomethyl)-2-aminoethansäure erreicht. Nach Zugabe der restlichen 100 g Lösemittel, Kristallisation, Filtration, Waschen und Trocknen wurden 49,1 g N-(Aminoiminomethyl)-2-aminoethansäure mit einem Gehalt von 99,1 % erhalten.

Das Pulverdiffraktogramm der erhaltenen nadeligen Kristalle zeigte die alleinige Anwesenheit von Form A an. Das zu CaCl₂ sehr ähnliche MgCl₂ bewirkt also nicht die Kristallisation von N-(Aminoiminomethyl)-2-aminoethansäure in Form B, obwohl die Löslichkeit von N-(Aminoiminomethyl)-2-aminoethansäure durch die Anwesenheit des Salzes ähnlich stark erhöht wird.

### Beispiel 5 (Vergleich) - Synthese von N-(Aminoiminomethyl)-2-aminoethansäure aus Glycin und Cyanamid in wässriger Lösung

112,6 g (1,5 mol) Glycin wurden in 300 g Wasser gelöst. Die Lösung wurde mit 21,6 g (0,27 mol) einer 50 %-igen Natronlauge versetzt wobei sich ein pH-Wert von 8,4 ergab. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 100,6 g N-(Aminoiminomethyl)-2-aminoethansäure mit einem Gehalt von 99,1 % erhalten. Die Ausbeute betrug 85,9 %.

Ein Pulverdiffraktogramm der erhaltenen fein-nadeligen Kristalle zeigte die alleinige Anwesenheit von Form A an.

### Beispiel 6 (erfindungsgemäß) - Synthese von N-(Aminoiminomethyl)-2-aminoethansäure aus Glycin und Cyanamid in einer 33 %-igen Calciumchloridlösung

Aus 100 g wasserfreiem Calciumchlorid und 200 g Wasser wurde eine Lösung hergestellt. Darin wurden 112,6 g (1,5 mol) Glycin gelöst und mit 21,6 g (0,27 mol) einer 50 %-igen Natronlauge ein pH-Wert von 8,4 eingestellt. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 99,3 g N-(Aminoiminomethyl)-2-aminoethansäure mit einem Gehalt von 99,2 % erhalten. Die Ausbeute betrug 84,8 %.

Ein Pulverdiffraktogramm der erhaltenen rundlichen Kristallaggregate aus radialstrahligen Einzelkristallen zeigte die alleinige Anwesenheit von Form B an. Beispiel 6a (erfindungsgemäß) - Synthese von N-(Aminoiminomethyl)-2-aminoethansäure aus Glycin und Cyanamid in einer 15 %-igen Calciumchloridlösung

Aus 45 g wasserfreiem Calciumchlorid und 255 g Wasser wurde eine Lösung hergestellt. Darin wurden 112,6 g (1,5 mol) Glycin gelöst und mit 21,5 g (0,27 mol) einer 50 %-igen Natronlauge ein pH-Wert von 8,4 eingestellt. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 99,6 g N-(Aminoiminomethyl)-2-aminoethansäure mit einem Gehalt von 99,3 % erhalten. Die Ausbeute betrug 84,5 %.

Ein Pulverdiffraktogramm der erhaltenen Kristalle zeigte, dass eine Mischung von Form A und Form B vorlag, wobei Form B den weitaus größeren Anteil ausmachte.

### Beispiel 6b (Vergleich) - Synthese von N-(Aminoiminomethyl)-2-aminoethansäure aus Glycin und Cyanamid in einer 1 %-igen Calciumchloridlösung

Aus 3 g wasserfreiem Calciumchlorid und 297 g Wasser wurde eine Lösung hergestellt. Darin wurden 112,6 g (1,5 mol) Glycin gelöst und mit 21,4 g (0,27 mol) einer 50 %-igen Natronlauge ein pH-Wert von 8,4 eingestellt. Bei 80 °C wurde im Verlauf von 4 Stunden eine Lösung von 42,04 g (1,0 mol) Cyanamid gelöst in 42 g Wasser zudosiert. Die Nachreaktion erfolgte für eine weitere Stunde bei 80 °C. Die erhaltene Suspension wurde auf 20 °C abgekühlt, abfiltriert, mit Wasser gewaschen und bei 60 °C getrocknet. Es wurden 100,1 g N-(Aminoiminomethyl)-2-aminoethansäure mit einem Gehalt von 99,2 % erhalten. Die Ausbeute betrug 84,8 %.

Ein Pulverdiffraktogramm der erhaltenen Kristalle zeigte, dass ausschließlich Form A vorlag.

Auch wenn N-(Aminoiminomethyl)-2-aminoethansäure durch Reaktion zwischen Glycin und Cyanamid generiert wird, lässt sich die anfallende Kristallform durch die Anwesenheit unterschiedlicher Konzentrationen von Calciumchlorid steuern.

### Beispiel 7 - Physikalisch-chemische Charakterisierung von N-(Aminoiminomethyl)-2-aminoethansäure der Form A und der Form B

### 7.1 Schmelz- bzw. Zersetzungspunkt

Zur Dynamischen Differential Scanning Calorimetry (DSC) wurde ein Gerät Mettler DSC 3+ mit 40 µl Aluminiumtiegel eingesetzt. Die Heizrate betrug 10 Kelvin pro Minute bei einem Temperaturbereich von 30 bis 350 °C. Jeweils ca. 1,4 mg der Produkte aus Beispiel 1 und 2 wurden in Aluminium-Tiegel eingewogen und bei Atmosphärendruck (960 mbar bei einer Höhenlage von 500 m über NN) vermessen.

Die Probe aus Beispiel 1 (= N-(Aminoiminomethyl)-2-aminoethansäure der Form A) zeigte einen onset (Wendepunkt der Schmelzkurve projiziert auf die Basislinie) von 280,5 °C und eine Peaktemperatur der Schmelzkurve von 286,3 °C. Die gesamte endotherme Schmelzwärme betrug 887 J/g. Das Produkt verfärbte sich beim Schmelzen von weiß nach braun.

Die Probe aus Beispiel 2 (= N-(Aminoiminomethyl)-2-aminoethansäure Form B) wurde anlog vermessen. Sie zeigte einen onset von 272,5 °C und einen Peak bei 280,4 °C, die Schmelzwärme betrug 860 J/g, die Verfärbung war identisch.

Form B schmilzt demnach ca. 6 bis 8 Kelvin tiefer als Form A und hat eine um 27 J/g niedrigere Schmelzwärme bzw. eine um 27 J/g höhere Gitterenergie. Anders ausgedrückt werden für Form B 27 J/g weniger Energie benötigt als für Form A, um den energiegleichen Schmelzzustand zu erreichen. Form B stellt damit eine metastabile Kristallform bzw. ein unter normalen Druck- und Temperaturbedingungen energetisch höherliegendes Polymorph von N-(Aminoiminomethyl)-2-aminoethansäure dar.

### 7.2 Bestimmung der Wasserlöslichkeit

100 g Wasser von 5 °C wurden vorgelegt. Darin wurde das Produkt aus Beispiel 1 (= N-(Aminoiminomethyl)-2-aminoethansäure Form A) bis zur Sättigung gelöst und die gelöste Menge durch Rückwägung bestimmt. Dann wurde die Temperatur auf 20 °C erhöht und so viel der Probe zugegeben, bis wieder der Sättigungspunkt erreicht war. Dasselbe wurde bei weiteren Temperaturen, maximal bei 95 °C, wiederholt. Eine analoge Messung wurde mit dem Produkt aus Beispiel 2 (= N-(Aminoiminomethyl)-2-aminoethansäure Form B) durchgeführt. Die erhaltenen Löslichkeitsdaten für beide Produkte wurden graphisch in Figur 6 zusammengefasst.

Beide Kristallformen von N-(Aminoiminomethyl)-2-aminoethansäure lösen sich mit steigenden Temperatur besser in Wasser. Die erfindungsgemäße N-(Aminoiminomethyl)-2-aminoethansäure Form B löst sich bei jeder Temperatur um ca. 20 % besser als die bekannte Form A.

### 7.3 Bestimmung der Dichte

Kristalle von N-(Aminoiminomethyl)-2-aminoethansäure Form A aus Beispiel 1 wurden bei 20 °C in Tetrachlormethan eingebracht, wo sie an der Oberfläche schwammen. Durch tropfenweiser Zugabe von Dichlormethan wurde die Dichte des flüssigen Mediums so lange erniedrigt, bis die Kristalle gerade eben in der Flüssigkeit zu schweben kamen, ohne aufzusteigen und ohne auf den Boden abzusinken. Die Dichte der flüssigen Phase wurde im Pyknometer bestimmt. Es wurden 1,50 g/cm³ gemessen.

Analog wurde mit Kristallen der Form B aus Beispiel 2 verfahren. Die Dichte bei 20 °C wurde zu 1,41 g/cm³ bestimmt.

Form B hat demnach eine um 6 % geringere Dichte als Form A. Dies korreliert mit der oben bestimmten niedrigeren Gitterenergie von Form B. Die gemessenen Kristalldichten stimmen zudem mit den aus den jeweiligen Gitterkonstanten berechneten Röntgenkristalldichten überein.

### 7.4 Bestimmung des Staubanteils

Das Produkt aus Beispiel 1 wurde über ein Sieb mit Maschenweite 63 µm (entspricht 230 Mesh - Mesh-Größe) abgesiebt. Es wurden 46 Gew.-% Feinanteil erhalten. Analog wurde mit der aus polygonalen, rundlichen Kristallaggregaten bestehenden Probe aus Beispiel 2 verfahren. Hier wurde ein Feinanteil von unter 3 Gew.-% bestimmt. Staubarme, damit sicher handhabbare Materialien sollten einen Staubanteil (d.h. Kornanteil < 63 µm) von unter 10 % aufweisen. Das Produkt aus Beispiel 2 (N-(Aminoiminomethyl)-2-aminoethansäure der Kristallform B) erfüllt dies, während das Vergleichsbeispiel 1 (N-(Aminoiminomethyl)-2-aminoethansäure der Kristallform A) dies nicht erfüllt.

### 7.5 Bestimmung des Schüttwinkels

Das Produkt aus Beispiel 1, bestehend aus ineinander verfilzten nadeligen Kristallen, wurde mit einer Vorrichtung nach DIN ISO 4324 durch einen Trichter auf eine ebene Fläche geschüttet. Nach Entfernen des Trichters wurde der Böschungswinkel des erhaltenen Kegels mit einer Winkelmesseinrichtung bestimmt. Dieser betrug ca. 45 °. N-(Aminoiminomethyl)-2-aminoethansäure Form A zeigt demnach ein schlechtes Fließverhalten. Das körnige Produkt aus Beispiel 2 wurde anlog vermessen. Hier wurde ein Böschungswinkel von ca. 25 ° erhalten. N-(Aminoiminomethyl)-2-aminoethansäure Form B zeigt demnach ein hervorragendes Fließverhalten.

### 7.6 Bestimmung der Schüttdichte

Eine eingewogene Menge des Produkts aus Beispiel 1 wurde in einen Messzylinder gegeben und durch zweimaliges festes Aufklopfen auf den Labortisch partiell verdichtet. Aus der Füllhöhe des Messzylinders wurde die Schüttdichte zu 0,37 g/cm³ bestimmt. Analog wurde mit dem Produkt aus Beispiel 2 verfahren. Hier wurde eine Schüttdichte von 0,62 g/cm³ bestimmt. N-(Aminoiminomethyl)-2-aminoethansäure der Form B hat somit eine deutlich erhöhte Schüttdichte, was für Verpackung, Transport und Handhabung des Produkts vorteilhaft ist.

### 7.7 Thermische Stabilität von N-(Aminoiminomethyl)-2-aminoethansäure Form B

a) N-(Aminoiminomethyl)-2-aminoethansäure Form B aus Beispiel 2 wurde für 6 Stunden bei 120 °C in den Trockenschrank gestellt. Anschließend wurde mittels Röntgen-Pulver-Diffraktometrie die Kristallform bestimmt. Diese blieb unverändert reine Kristallform B.
b) N-(Aminoiminomethyl)-2-aminoethansäure Form B aus Beispiel 2 wurde mit 20 % Wasser angefeuchtet, für 6 Stunden bei 65 °C in einem geschlossenen Gefäß inkubiert, dann getrocknet. Das Röntgen-Pulver-Diffraktogramm zeigte keinerlei Veränderung, Form B blieb stabil.
c) N-(Aminoiminomethyl)-2-aminoethansäure Form B aus Beispiel 2 wurde zu einer 10 %-igen Suspension in Wasser angesetzt. Diese Suspension wurde 2 Stunden bei 80 °C gerührt. Dann wurde abgekühlt, der Feststoff abfiltriert und getrocknet. Die Röntgen-Pulver-Diffraktometrie ergab, dass ein Gemisch aus Kristallform A und B vorlag.
d) N-(Aminoiminomethyl)-2-aminoethansäure Form B aus Beispiel 2 wurde bei 80 °C in Wasser gelöst, durch Abkühlen der Lösung großteils wieder auskristallisiert, abfiltriert und getrocknet. Die Röntgen-Pulver-Diffraktometrie ergab reine Kristallform A.

N-(Aminoiminomethyl)-2-aminoethansäure Form B ist also in fester Form sehr beständig, hat jedoch die Tendenz, über die wässrige Lösung in Kristallform A überzugehen. Auch dieses Verhalten bestätigt die metastabile Kristallstruktur von Form B.

### Beispiel 8 - Synthese N-(Aminoiminomethyl)-2-aminoethansäure gemäß Stand der Technik in dem Calcium zugegen ist - DE 964 590 B

Anmerkung: Der gemäß DE 964 590 B eingesetzte Kalkstickstoff hat nur einen Gehalt von 53 %; dies entspricht 15,9 % N. In dem folgenden Beispiel wurde Kalkstickstoff mit einem Gehalt von 68,6 % eingesetzt; dies entspricht 24 % N. Die Einsatzmenge wurde entsprechend angepasst.

154,5 g technisches Calciumcyanamid mit einem Gehalt von 68,6 % CaNCN wurden in 800 g Wasser suspendiert. Bei 20 °C wurde eine Mischung aus 191,6 g einer 96 %igen Schwefelsäure und 300 g Wasser zudosiert, wobei Cyanamid in Lösung ging und Calciumsulfat aus der Lösung ausfiel und ein pH-Wert von 7,5 erhalten wurde. Calciumsulfat und sonstige unlösliche Bestandteile wurden abfiltriert und das Filtrat mit wenig Schwefelsäure auf pH 4,9 gestellt. Die erhaltene Lösung wurde unter vermindertem Druck bei ca. 10 mbar auf ein Gesamtvolumen von 200 cm³ eingedampft. Weiteres ausgefallenes Calciumsulfat wurde abfiltriert. Die erhaltene wässrige Cyanamid-Löung hatte einen Cyanamid-Gehalt von 26,4 % und einen Calciumgehalt von 0,56 g/Liter. (Anmerkung: entspricht einer Cyanamid-Ausbeute von 95 % und der Gips-Löslichkeit von 2,4 g/l).

Diese Cyanamid-Lösung wurde mit 30 g Glycin versetzt und mit 19,8 g einer 50 %-igen wässrigen Natronlauge auf pH 9,4 gestellt. Das Reaktionsgemisch wurde für 1,5 Stunden auf 95 °C erhitzt und dann über Nacht auf Raumtemperatur abgekühlt. Ausgefallene N-(Aminoiminomethyl)-2-aminoethansäure sowie mitentstandenes Dicyandiamid wurden abfiltriert, der Filterrückstand in 180 g Wasser aufgenommen, 2 Stunden bei 50 °C ausgelaugt, bei 50 °C filtriert und mit Wasser gewaschen. Nach Trocknung bei 60 °C wurden 38,4 g N-(Aminoiminomethyl)-2-aminoethansäure erhalten. Die Ausbeute betrug 82 % bezogen auf eingesetztes Glycin.

Eine Röntgen-Pulverdiffraktometrie ergab, dass N-(Aminoiminomethyl)-2-aminoethansäure ausschließlich in der Kristallform A entstanden war.

## Patentansprüche

1. Thermodynamisch metastabile Kristallmodifikation von N-(Aminoiminomethyl)-2-aminoethansäure, **dadurch gekennzeichnet, dass** die Kristallmodifikation im Röntgen-Pulver-Diffraktogramm der Kristallmodifikation bei Verwendung von Cu-Kα₁-Strahlung die stärksten Reflexbanden bei 2Θ = 20,2 ° und 23,3 ° und 23,8 ° und 25,3 ° bei einer Messgenauigkeit von +/- 0,2 ° zeigt.

2. Kristallmodifikation nach Anspruch 1, **dadurch gekennzeichnet, dass** die Kristallmodifikation die orthorhombische Raumgruppe P2₁2₁2₁ mit Z = 8 mit den Gitterkonstanten a = 7,7685 Å, b = 7,7683 Å und c = 17,4261 Å bei 105 Kelvin und einer Messgenauigkeit von +/- 0,001 Å aufweist.

3. Kristallmodifikation nach Anspruch 2, **dadurch gekennzeichnet, dass** die Kristallmodifikation ein Zellvolumen von 1052 Å³ bei 105 Kelvin aufweist.

4. Kristallmodifikation nach Anspruch 1 oder 2, **dadurch gekennzeichnet, dass** die Kristallmodifikation eine experimentelle Kristalldichte von 1,41 g/cm³ +/- 0,03 g/cm³ bei 20 °C aufweist.

5. Kristallmodifikation nach einem der zuvor genannten Ansprüche, **dadurch gekennzeichnet, dass** die Kristallmodifikation eine endotherme Schmelzwärme im Bereich von 850 bis 870 J/g aufweist.

6. Kristallmodifikation nach einem der zuvor genannten Ansprüche, **dadurch gekennzeichnet, dass** die Kristallmodifikation einen Zersetzungspunkt im Bereich von 270 bis 275 °C aufweist.

7. Verfahren zur Herstellung einer thermodynamisch metastabilen Kristallmodifikation von N-(Aminoiminomethyl)-2-aminoethansäure gemäß einem der Ansprüche 1 bis 6, **dadurch gekennzeichnet, dass** N-(Aminoiminomethyl)-2-aminoethansäure aus einer Lösung enthaltend 5 bis 50 Gew.-% Calciumchlorid kristallisiert wird.

8. Verfahren nach Anspruch 7, **dadurch gekennzeichnet, dass** N-(Aminoiminomethyl)-2-aminoethansäure aus einer Lösung enthaltend 10 bis 40 Gew.-% Calciumchlorid kristallisiert wird.

9. Verfahren nach Anspruch 7 oder 8, **dadurch gekennzeichnet, dass** N-(Aminoiminomethyl)-2-aminoethansäure in einem Temperaturbereich von -40 bis 100 °C kristallisiert wird.

10. Verfahren nach einem der zuvor genannten Ansprüche 7 bis 9, **dadurch gekennzeichnet, dass** N-(Aminoiminomethyl)-2-aminoethansäure mit einer Abkühlrate im Bereich von 0,01 bis 5 K/min in einem Temperaturbereich von -40 bis 100 °C kristallisiert wird.

11. Verfahren nach einem der zuvor genannten Ansprüche 7 bis 10, **dadurch gekennzeichnet, dass** die Lösung als Lösemittel ein Lösemittel aus der Gruppe Wasser, Alkohole, Ester, Nitrile, Ketone oder Mischungen hiervon enthält.

12. Verwendung der thermodynamisch metastabilen Kristallmodifikation von N-(Aminoiminomethyl)-2-amino-ethansäure gemäß einem der Ansprüche 1 bis 6 zur Herstellung eines Futtermittelzusatzes.

13. Futtermittelzusatz umfassend eine thermodynamisch metastabile Kristallmodifikation von einem der Ansprüche 1 bis 6.

## Claims

1. Thermodynamically metastable crystal modification of N-(aminoiminomethyl)-2-aminoacetic acid, **characterised in that** the crystal modification in the x-ray powder diffractogram of the crystal modification shows the strongest reflex bands at 2Θ = 20.2° and 23.3° and 23.8° and 25.3° at a measuring accuracy of +/- 0.2° when using Cu-Kα₁ radiation.

2. Crystal modification according to claim 1, **characterised in that** the crystal modification has the orthorhombic space group P2₁2₁2₁ with Z = 8 with the lattice constants a = 7.7685 Å, b = 7.7683 Å and c = 17.4261 Å at 105 Kelvin and a measuring accuracy of +/- 0.001 Å.

3. Crystal modification according to claim 2, **characterised in that** the crystal modification has a cell volume of 1052 Å³ at 105 Kelvin.

4. Crystal modification according to claim 1 or 2, **characterised in that** the crystal modification has an experimental crystal density of 1.41 g/cm³ +/- 0.03 g/cm³ at 20°C.

5. Crystal modification according to one of the above mentioned claims, **characterised in that** the crystal modification has an endothermic melt heat within a range of 850 to 870 J/g.

6. Crystal modification according to one of the above mentioned claims, **characterised in that** the crystal modification has a decomposition point within a range of 270 to 275°C.

7. Method for producing a thermodynamically metastable crystal modification of N-(aminoiminomethyl)-2-aminoacetic acid according to one of claims 1 to 6, **characterised in that** N-(aminoiminomethyl)-2-aminoacetic acid is crystallised from a solution containing 5 to 50 wt.% calcium chloride.

8. Method according to claim 7, **characterised in that** N-(aminoiminomethyl)-2-aminoacetic acid is crystallised from a solution containing 10 to 40 wt.% calcium chloride.

9. Method according to claim 7 or 8, **characterised in that** N-(aminoiminomethyl)-2-aminoacetic acid is crystallised within a temperature range of -40 to 100°C.

10. Method according to one of the above mentioned claims 7 to 9, **characterised in that** N-(aminoiminomethyl)-2-aminoacetic acid is crystallised with a cooling rate within a range of 0.01 to 5 K/min within a temperature range of -40 to 100°C.

11. Method according to one of the above mentioned claims 7 to 10, **characterised in that** the solution contains a solvent from the group of water, alcohols, esters, nitriles, ketones or mixture of the same as a solvent.

12. Use of the thermodynamically metastable crystal modification of N-(aminoiminomethyl)-2-aminoeacetic acid according to one of the claims 1 to 6 for producing an animal feed additive.

13. Animal feed additive comprising a thermodynamically metastable crystal modification of one of the claims 1 to 6.

## Revendications

1. Modification cristalline thermodynamiquement métastable de l'acide N-(aminoiminométhyl)-2-aminoéthanoïque, **caractérisée en ce que** la modification cristalline présente dans le diffractogramme de poudre aux rayons X de la modification cristalline, en utilisant un rayonnement Cu-Kα1, les bandes de réflexion les plus fortes à 2Θ = 20,2° et 23,3 ° et 23,8 ° et 25,3 ° avec une précision de mesure de +/- 0,2°.

2. Modification cristalline selon la revendication 1, **caractérisée en ce que** la modification cristalline présente le groupe spatial orthorhombique P2₁2₁2₁, avec Z = 8 avec les constantes de réseau a = 7,7685 Å, b = 7,7683 Å et c = 17,4261 Å à 105 Kelvin et une précision de mesure de +/- 0,001 Å.

3. Modification cristalline selon la revendication 2, **caractérisée en ce que** la modification cristalline présente un volume cellulaire de 1052 Å³ à 105 Kelvin.

4. Modification cristalline selon la revendication 1 ou 2, **caractérisée en ce que** la modification cristalline présente une densité cristalline expérimentale de 1,41 g/cm³ +/- 0,03 g/cm³ à 20 °C.

5. Modification cristalline selon l'une des revendications susmentionnées, **caractérisée en ce que** la modification cristalline présente une chaleur de fusion endothermique dans la plage de 850 à 870 J/g.

6. Modification cristalline selon l'une des revendications susmentionnées, **caractérisée en ce que** la modification cristalline présente un point de décomposition dans la plage de 270 à 275 °C.

7. Procédé de préparation d'une modification cristalline thermodynamiquement métastable de l'acide N-(aminoiminornéthyl)-2-aminoéthanoïque selon l'une des revendications 1 à 6, **caractérisé en ce que** l'acide N-(aminoiminométhyl)-2-aminoéthanoïque est cristallisé à partir d'une solution contenant 5 à 50 % en poids de chlorure de calcium.

8. Procédé selon la revendication 7, **caractérisé en ce que** l'acide N-(aminoiminométhyl)-2-aminoéthanoïque est cristallisé à partir d'une solution contenant 10 à 40 % en poids de chlorure de calcium.

9. Procédé selon la revendication 7 ou 8, **caractérisé en ce que** l'acide N-(aminoiminométhyl)-2-aminoéthanoïque est cristallisé dans une plage de température de -40 à 100 °C.

10. Procédé selon l'une des revendications 7 à 9 susmentionneés, **caractérisé en ce que** l'acide N-(aminoiminométhyl)-2-aminoéthanoïque est cristallisé à une vitesse de refroidissement comprise entre 0,01 et 5 K/min dans une plage de température de - 40 à 100 °C.

11. Procédé selon l'une des revendications 7 à 10 susmentionneés, **caractérisé en ce que** la solution contient comme solvant un solvant choisi parmi l'eau, les alcools, les esters, les nitriles, les cétones ou des mélanges de ceux-ci.

12. Utilisation de la modification cristalline thermodynamiquement métastable de l'acide N-(aminoiminométhyl)-2-aminoéthanoïque selon l'une des revendications 1 à 6 pour la préparation d'un additif alimentaire pour animaux.

13. Additif alimentaire pour animaux, comprenant une modification cristalline thermodynamiquement métastable selon l'une des revendications 1 à 6.
